# EUROPEAN PATENT APPLICATION

(11) **EP 3 536 316 A1**
(43) Date of publication of application: **11.09.2019**
(21) Application number: 17866642.6
(22) Date of filing: 30.10.2017
(51) Int. Cl.: A61K 31/20, A41B 17/00, A61K 8/36, A61K 8/37, A61K 31/201, A61K 31/23, A61K 31/231, A61K 31/575, A61P 17/10, A61P 31/04, A61Q 19/00

(54) **ACNE STRAIN-SELECTIVE ANTIBACTERIAL AGENT**

(30) Priority: 01.11.2016 JP 2016214239
(71) Applicant: Momotani Juntenkan Co., Ltd., Osaka-shi, Osaka 552-0012 (JP); Osaka Research Institute of Industrial Science and Technology, Izumi-shi, Osaka 594-1157 (JP)
(72) Inventor: UYAMA, Ayaka, Osaka-shi Osaka 552-0012 (JP); SUGINO, Teizo, Osaka-shi Osaka 552-0012 (JP); NAGAO, Toshihiro, Osaka-city Osaka 536-8553 (JP); TANAKA, Shigemitsu, Osaka-city Osaka 536-8553 (JP)
(74) Representative: Kuhnen & Wacker Patent- und Rechtsanwaltsbüro PartG mbB
(86) International application number: PCT/JP2017/039144
(87) International publication number: WO 2018/084112

(57) **Abstract**

Provide is a medicinal agent having a strain-selective antibacterial activity for acne bacteria which are indigenous bacteria in the human skin, namely having a stronger bactericidal or growth suppressing activity on an acne bacterium strain enriched in an acne patient group rather than on an acne bacterium strain enriched in a healthy subject group.

An acne strain-selective antibacterial agent, comprising at least one fatty acid selected from the group consisting of saturated fatty acids having 14 to 20 carbon atoms (C14:0 to C20:0) and a monounsaturated fatty acid having 18 carbon atoms (C18:1) in a free form or a salt form, or an ester thereof.

## Description

### Technical Field

The present invention relates to an acne strain-selective antibacterial agent, and a composition containing the antibacterial agent.

### Description of the Background

Acne vulgaris (hereinafter referred to as "acne") is an inflammatory disease found in the hair follicle or the sebaceous gland in the face, chest or back, which is believed to develop according to a complicated mechanism involving disorder of lipid metabolism (endocrine factor), dyskeratosis, and growth of bacteria including *Propionibacterium acnes* (hereinafter referred to as "acne bacterium"). It is said that acne generally develops when firstly pores secreting sebum are clogged therewith, and accumulated sebum in the pores are degraded by a lipase from acne bacteria to generate an excessive amount of free fatty acids, which are then oxidized to cause inflammation.

Acne usually develops at or after puberty and it is said that more than 90% of humans experience it. However, since it remits naturally at or after adolescence, it is regarded as a physiological phenomenon rather than a disease. It is a recent trend that acne frequently appearing in the face is actively treated due to growing beauty consciousness.

Currently, there are two types of acne treatments, namely a treatment by a medical institution and a treatment with a cosmetic. In the case of a treatment by a medical institution an external medicine such as adapalene for controlling cornification of an epidermal keratinocyte, or an external medicine or an internal medicine of an antibiotic, which kills acne bacteria, is used according to the acne vulgaris clinical practice guideline (The Japanese Dermatological Association). In the case of a treatment with a cosmetic, an anti-inflammatory agent (such as glycyrrhizinic acid), a keratin releasing agent (such as salicylic acid), a bactericidal agent (such as benzoic acid, isopropylmethylphenol, or homosulfamine), and a sebum secretion regulating agent (such as ethyl estradiol) are used in combination.

In recent years, phylogenetic analyses of the acne bacterium have been performed, and it has become clear that there are an acne bacterium strain enriched in a healthy subject group rather than in an acne patient group, and an acne bacterium strain contrarily enriched in an acne patient group rather than in a healthy subject group (Non Patent Literatures 1 to 3 and Patent Literature 1).

It is becoming clear that an indigenous microbial flora of the human skin plays an important role in skin health and disease. A selective antibacterial agent capable of decreasing the ratio of *Staphylococcus aureus* (a bacterium undesirable to the skin) causing aggravation of atopic dermatitis to the total staphylococcus, and increasing the ratio of *Staphylococcus epidermidis* (a bacterium beneficial to the skin) to the total staphylococcus is disclosed with respect to staphylococcus as an indigenous bacterium of the skin (Non Patent Literature 4).

### Citation List

### Patent Literature

Patent Literature 1
National Publication of International Patent Application No. 2015-512255

### Non Patent Literature

Non Patent Literature 1
   Tomida, S. et al., MBio, 4, e00003-e00013, 2013
Non Patent Literature 2
   A. McDowell et al., PLoS ONE, 7, e41480, 2012
Non Patent Literature 3
   S. Fitz-Gibbon et al., J. Investigative Dermatology, 133, 2152, 2013
Non Patent Literature 4
   Nagao et al., The Japan Society for Bioscience, Biotechnology and Agrochemistry 2016 Annual Meeting, Poster No. 3F-200

### Summary

When a bactericidal agent or an antibiotic, which has such a strong bactericidal action as to be able to eliminate all the indigenous microbial flora of the skin, is used, there are risks that bacteria having a beneficial effect on the skin may also be eliminated, and emergence and proliferation of a bacterium resistant to the medicinal agent may be promoted. There is also a risk of preferential growth of a bacterium undesirable for the skin, when the use of the bactericidal agent or the antibiotic is discontinued. In a case where the selective antibacterial agent as disclosed in Non Patent Literature 4 is used, even when its use is discontinued, it may be possible to avoid the risk of preferential growth of a bacterium undesirable for the skin, because a bacterial flora in which the proportion of bacteria having a beneficial effect on the skin is enhanced may exist in the skin.

It is said that acne bacteria have a role of suppressing invasion or growth of pathogenic bacteria by degrading sebum into glycerol and a fatty acid to keep the skin weakly acidic. Therefore, it is undesirable to apply, to the skin, a bactericidal agent or an antibiotic having such a bactericidal action as to be able to eliminate the entire acne bacteria.

In the field related to the prevention and treatment of acne, there is a demand for a medicinal agent having a strain-selective antibacterial activity for acne bacteria which are indigenous bacteria in the human skin, namely having a stronger bactericidal or growth suppressing activity on an acne bacterium strain enriched in an acne patient group rather than on an acne bacterium strain enriched in a healthy subject group.

The present inventors conducted intensive studies on a wide variety of substances including medicinal agents which have been commonly used as a bactericidal agent or an antibiotic for skin, and as a result found that a specific fatty acid or an ester thereof has a selective growth suppression action or bactericidal action on an acne bacterium strain enriched in an acne patient group, thereby completing the present invention.

The present invention provides the following acne strain-selective antibacterial agent and a composition comprising the antibacterial agent.

The first aspect of the present invention relates to an acne strain-selective antibacterial agent comprising at least one fatty acid selected from the group consisting of saturated fatty acids having 14 to 20 carbon atoms (C14:0 to C20:0) and a monounsaturated fatty acid having 18 carbon atoms (C18:1) in a free form or a salt form, or an ester thereof.

The second aspect of the present invention relates to use of at least one fatty acid selected from the group consisting of saturated fatty acids having 14 to 20 carbon atoms (C14:0 to C20:0) and a monounsaturated fatty acid having 18 carbon atoms (C18:1) in a free form or a salt form, or an ester thereof, for producing an acne strain-selective antibacterial agent or a skin-care composition.

With an acne strain-selective antibacterial agent according to the present invention, the growth of an acne bacterium strain enriched in an acne patient group may be suppressed, so as to enhance the proportion of an acne bacterium strain enriched in a healthy subject group in the total acne bacteria. As a result, skin diseases including acne may be prevented or treated. Further, owing to the presence of an indigenous microbial flora in the skin including the acne bacterium strain enriched in the healthy subject group, growth of bacteria that can adversely affect the skin environment can be suppressed to maintain a favorable skin environment.

### Brief Description of Drawings

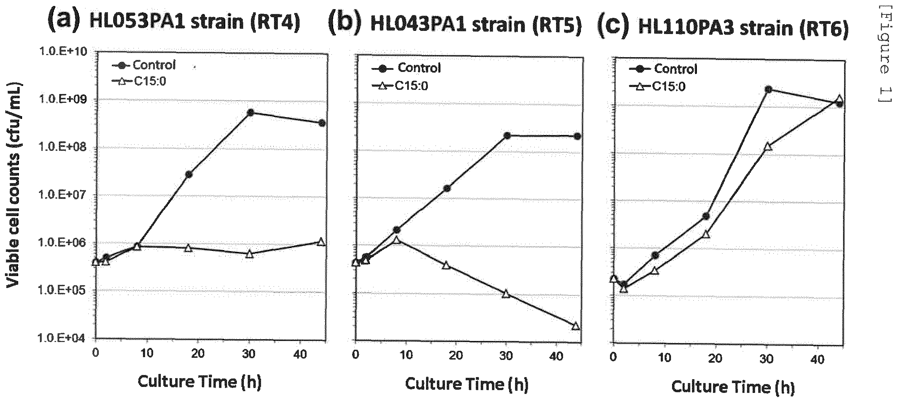

Figure 1 is line graphs showing the time courses of the viable cell counts of acne bacterium strains (a: HL053PA1 strain (RT4 strain), b: HL043PA1 strain (RT5 strain), and c: HL110PA3 strain (RT6 strain)) in the presence (C15:0) and the absence (control) of a saturated fatty acid.

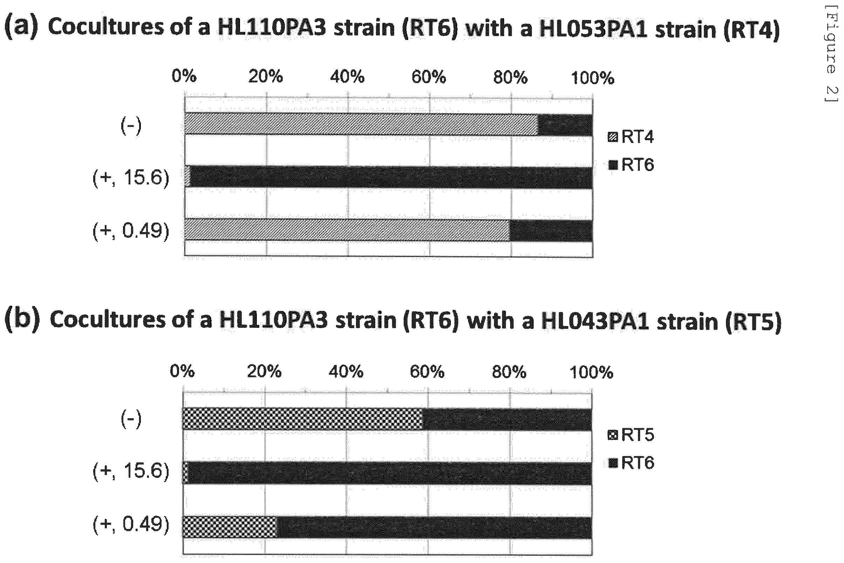

Figure 2 is band graphs showing the portions of respective bacterium strains for the acne bacterium strains after cocultures of a dominant strain in healthy subjects (HL110PA3 strain (RT6 strain)) with a dominant strain in acne patients (a: HL053PA1 strain (RT4 strain), or b: HL043PA1 strain (RT5 strain)) in the presence (+) and the absence (-) of a saturated fatty acid (C15:0). The saturated fatty acid (C15:0) was used at 15.6 µg/mL or 0.49 µg/mL.

### Embodiments

*"Propionibacterium acnes"* or "acne bacterium" is herein a bacterium included in a skin bacterial flora. The acne bacterium is, as described in Non Patent Literatures 1 to 3 and Patent Literature 1, classified based on 10 kinds of specific 16S ribosomal DNA sequences (also referred to as "ribotypes (RT)". The ribotype of an acne bacterium strain enriched in a healthy subject group rather than in an acne patient group has been identified as "RT4" or "RT5", and the ribotype of an acne bacterium strain enriched in an acne patient group rather than in a healthy subject group has been identified as "RT6" (Non Patent Literatures 1 to 3, and Patent Literature 1). Separately, the acne bacterium has been also classified based on a single nucleotide substitution (SNV) (McDowell, A. et al., PLoS One, 7: e41480, 2012).

A "dominant strain in acne patients" means herein among acne bacteria an acne bacterium strain enriched in an acne patient group rather than in a healthy subject group. The term of dominant strain in acne patients may be used hereunder interchangeably with an acne bacterium strain whose ribotype is "RT4" or "RT5" (RT4 strain, or RT5 strain). A "dominant strain in healthy subjects" means herein among acne bacteria an acne bacterium strain enriched in a healthy subject group rather than in an acne patient group. The term of dominant strain in healthy subjects may be used hereunder interchangeably with an acne bacterium strain whose ribotype is "RT6" (RT6 strain).

An "antibacterial agent" represents herein a reagent for killing microorganisms, or suppressing or inhibiting the growth or proliferation of microorganisms.

An "acne strain-selective antibacterial agent" represents herein an antibacterial agent which has an acne strain-selective antibacterial activity described below, and comprises at least one fatty acid selected from the group consisting of saturated fatty acids having 14 to 20 carbon atoms (C14:0 to C20:0) and a monounsaturated fatty acid having 18 carbon atoms (C18:1) in a free form or a salt form, or an ester thereof.

An "ester" of a fatty acid represents herein a compound which is formed by dehydration condensation of a carboxyl group of a fatty acid according to the present invention with a hydroxy group of glycerol, cholesterol, an aliphatic alcohol, or the like, and examples thereof include, but not limited to, a monoacylglycerol, a diacylglycerol, a triacylglycerol, a cholesterol ester, a wax ester, and a mixture thereof. In an embodiment, the ester may be a monoacylglycerol, a diacylglycerol, a cholesterol ester, a wax ester, or a mixture thereof. In another embodiment, the ester may be a monoacylglycerol, a diacylglycerol, or a mixture thereof, and preferably it is a monoacylglycerol, or it contains a monoacylglycerol.

Herein, "acne strain-selective antibacterial activity" or "strain-selective antibacterial activity" means an index indicating better inhibition of growth of a dominant strain in acne patients compared to that of a dominant strain in healthy subjects. It is expressed by the average value of the values (MIC_{RT6}/MIC_{RT4}, and MIC_{RT6}/MIC_{RT5}) obtained by dividing the minimum inhibitory concentration (MIC) of an object substance with respect to the dominant strain in healthy subjects (RT6 strain) (MIC_{RT6}) respectively by the MIC of the object substance with respect to the dominant strain in acne patients (RT4 strain) (MIC_{RT4}), and by the MIC of the object substance with respect to the dominant strain in acne patients (RT5 strain) (MIC_{RT5}).

An MIC is measured herein according to the broth microdilution method which is the standard method of Japanese Society of Chemotherapy.

In an embodiment, the strain-selective antibacterial activity is the arithmetic average of a value (MIC_{RT6}/MIC_{RT4}) obtained by dividing MIC_{RT6} of an object substance with respect to the HL110PA3 strain (dominant strain in healthy subjects: RT6 strain) by MIC_{RT4} of the object substance with respect to the HL053PA1 strain (dominant strain in acne patients: RT4 strain), and a value (MIC_{RT6}/MIC_{RT5}) obtained by dividing the MIC_{PT6} by MIC_{RT5} of the object substance with respect to the HL043PA1 strain (dominant strain in acne patients: RT5 strain), which can be obtained by using the measuring method described in Example 1 below or an equivalent method thereof. The equivalent measuring method of the measuring method described in Example 1 means a measuring method substantially the same as in Example 1 except that the measuring condition may be changed corresponding to the product characteristics (for example, pH) of an acne strain-selective antibacterial agent according to the present invention, or a composition containing the same. As an example of such a measuring method, the measuring method described in Example 2 may be referred to.

As an example, the fatty acid according to the invention is at least one selected from the group consisting of myristic acid (C14:0), pentadecanoic acid (C15:0), palmitic acid (C16:0), heptadecanoic acid (C17:0), stearic acid (C18:0), oleic acid (C18:1), vaccenic acid (C18:1), and arachidic acid (C20:0). In an embodiment, an acne strain-selective antibacterial agent may contain a single or two or more kinds of fatty acids, or esters thereof according to the present invention in a free form or a salt form.

An acne strain-selective antibacterial agent according to the first aspect of the present invention may be appropriately selected from the aforedescribed fatty acids corresponding to the application environment (for example, pH), or the type or formulation form of a composition or product to be prepared using the same. It is preferable that a fatty acid according to the present invention has a high strain-selective antibacterial activity, namely the MIC against a dominant strain in acne patients and the MIC against a dominant strain in healthy subjects are discrepant from each other as much as possible. The strain-selective antibacterial activity of the fatty acid in a composition or product containing the same, or in an application environment may be more than 2, is preferably more than 6, more preferably more than 20, and further preferably more than 60.

In an example, the fatty acid according to the present invention is at least one selected from the group consisting of myristic acid, pentadecanoic acid, palmitic acid, heptadecanoic acid, stearic acid, and oleic acid. In another example, the fatty acid is at least one selected from the group consisting of myristic acid, pentadecanoic acid, palmitic acid, heptadecanoic acid, and stearic acid. In another example, the fatty acid is at least one selected from the group consisting of myristic acid, pentadecanoic acid, palmitic acid, and heptadecanoic acid. In another example, the fatty acid is at least one selected from the group consisting of myristic acid, pentadecanoic acid, and palmitic acid.

Since the fatty acid according to the present invention is capable of exhibiting stably a high strain-selective antibacterial activity, for example, in a broad pH range from weakly acidic to weakly alkaline (e.g. pH 3 to 12, preferably pH 5 to 12), and preferably in a pH range from weakly acidic to nearly neutral (e.g. pH 3 to 7, preferably pH 5 to 7), it contains at least one selected from the group consisting of myristic acid, pentadecanoic acid, and heptadecanoic acid. Preferably, the fatty acid contains at least one selected from the group consisting of myristic acid, and heptadecanoic acid. More preferably, the fatty acid contains at least pentadecanoic acid, or contains substantially only pentadecanoic acid. As another example, the fatty acid contains at least one selected from the group consisting of myristic acid and palmitic acid, and the fatty acid may further contain pentadecanoic acid.

Since the fatty acid according to the present invention is capable of exhibiting a high strain-selective antibacterial activity, for example, in a pH range from nearly neutral to weakly alkaline (e.g. pH 7 to 12), it contains at least one selected from the group consisting of pentadecanoic acid, palmitic acid, heptadecanoic acid, stearic acid, oleic acid, vaccenic acid, and arachidic acid. The fatty acid preferably contains at least one selected from the group consisting of pentadecanoic acid, palmitic acid, heptadecanoic acid, stearic acid, and oleic acid. More preferably, the fatty acid contains at least one selected from the group consisting of pentadecanoic acid, and heptadecanoic acid. Further preferably, the fatty acid contains at least pentadecanoic acid, or contains substantially only pentadecanoic acid.

There is no particular restriction on the fatty acid according to the present invention, insofar as it meets the specifications acceptable for blend in pharmaceuticals, quasi-drugs, cosmetics, or clothing. The fatty acid or an ester thereof according to the present invention may be a purified extract from a natural source. Although there is no particular restriction on the natural source, examples thereof include a vegetable source, such as rapeseed and palm, and an animal source, such as fish egg and an animal oil. As a fatty acid or an ester thereof according to the present invention, a commercial product may be used as it is, or further purified and used, or it may be chemically synthesized from a commercial product. For an example, a monoacylglycerol according to the present invention may be prepared from a commercially available fatty acid according to the method described in Example.

There is no particular restriction on the formulation form of the antibacterial agent, and it may be a solution system, a solubilized system, an emulsion system, a powdered system, a gel system, an ointment system, a cream, a mist, an aerosol, a water-oil two-phase system, or a water-oil-powder three-phase system.

In an embodiment, the antibacterial agent may contain the fatty acid or an ester thereof as appropriate corresponding to its formulation form. For example, the antibacterial agent contains 0.01 to 99.9 wt% of the fatty acid or an ester thereof in terms of a free form with respect to the total weight of the agent, for example 0.1 to 99 wt%, 1 to 98 wt%, 10 to 95 wt%, 20 to 95 wt%, 40 to 95 wt%, or 60 to 95 wt%, or alternatively 1 to 80 wt%, 1 to 50 wt%, or 10 to 50 wt%.

The antibacterial agent may appropriately blend according to need one or more kinds of other ingredients commonly used in an antibacterial agent, such as a watersoluble polymer, a sequestering agent, a lower alcohol, a polyhydric alcohol, a saccharide, an amino acid, an organic amine, a pH adjuster, a vitamin, an antioxidant, and a preservative, to the extent that the acne strain-selective antibacterial activity is not inhibited or eliminated.

There is no particular restriction on the method for preparing the antibacterial agent, and it may be appropriately prepared corresponding to the formulation form using a method known in the art. For example, in the case of a liquid preparation, it may be prepared by, but not limited to, adding the fatty acid or an ester thereof in a free form or a salt form according to the present invention, together with other ingredients into an appropriate solvent (for example, a lower alcohol such as ethanol, or DMSO), followed by mixing.

The antibacterial agent is used at an "effective dose", at which a severe side effect does not appear, and the growth of at least one dominant strain in acne patients (for example, HL053PA1 strain (RT4 strain) and/or HL043PA1 strain (RT5 strain)) can be selectively suppressed or inhibited. An "effective dose" can be appropriately set by those skilled in the art, and, for example, may be appropriately adjusted based on the MIC disclosed hereunder as a guide. As the fatty acid, that having a high strain-selective antibacterial activity and exhibiting a low MIC against a dominant strain in acne patients is preferable from the economical point of view, because its blending amount can be at a low level.

As the fatty acid, for example, in the pH range tested in the following Example, C14:0 to C18:0 for each of which the MIC against a dominant strain in acne patients (RT4 strain and/or RT5 strain) was 250 µg/mL are preferable from the viewpoint of blending amount, C15:0 to C17:0 for each of which the MIC was 125 µg/mL or less are more preferable, and C15:0 or C17:0 for each of which the MIC was 62.5 µg/mL or less is further preferable. Further preferably, the fatty acid is C15:0 for which the MIC was 15.6 µg/mL or less.

In the second aspect, the present invention provides a skin-care composition comprising at least one fatty acid selected from the group consisting of saturated fatty acids having 14 to 20 carbon atoms (C14:0 to C20:0) and a monounsaturated fatty acid having 18 carbon atoms (C18:1) in a free form or a salt form, or an ester thereof, and a carrier acceptable for skin application. The features relating to a fatty acid or an ester thereof described in the first aspect apply also to the fatty acid in this aspect. In an embodiment, the composition comprises the acne strain-selective antibacterial agent and a carrier acceptable for skin application.

A "carrier acceptable for skin application" means herein a carrier, with which an antibacterial agent or a composition is highly safe, has excellent temporal stability, and can be applied to the skin (for example, by establishing contact with the skin) without an irritation (for example odor) and/or an allergic reaction. Such a carrier may be appropriately selected corresponding to the form of the composition, or the purpose of use, and examples thereof include, but not limited to, any of water (such as ion exchanged water, and rose water), an oil (such as a higher alcohol and a silicone oil), an alcohol (such as ethanol and isopropanol), and a combination thereof. In an embodiment, the composition contains 0.001 to 3 wt% of the fatty acid or an ester thereof in terms of a free form with respect to the total weight of the composition. In another embodiment, the composition contains the fatty acid or an ester thereof in terms of a free form for example at 0.01 to 5 wt%, 0.01 to 3 wt%, 0.01 to 1 wt%, or 0.1 to 1 wt% with respect to the total weight of the composition. In another embodiment, the composition is substantially free from a fatty acid other than the fatty acids. The expression of "substantially free from a fatty acid other than the fatty acid according to the present invention" may mean herein that a fatty acid other than the fatty acid according to the present invention may be contained at less than 0.1 wt%, less than 0.05 wt%, or less than 0.01 wt% with respect to the total weight of the composition.

In an embodiment, the composition is for treatment or prevention of acne. The term "treat" or "treatment" refers herein to an action to partially or completely palliate, ameliorate, and/or cure at least one of a symptom, a sign, progression, or recurrence of a target skin disease (for example, acne) or symptoms (for example, appearance). The composition contains, for example, the above-mentioned fatty acid or an ester thereof, or the acne strain-selective antibacterial agent according to the first aspect at a dose effective for treating or preventing acne.

The "dose effective for treatment" of the fatty acid or an ester thereof, or the antibacterial agent is a dose sufficient to cure or at least partially arrest a disease or its complication in a subject already suffering from skin symptoms (for example, acne). The dose effective for treatment may vary depending on the severity of the symptom to be treated and the subject's particulars (such as gender and age). The "dose effective for prevention" of the antibacterial agent is a dose sufficient to ameliorate the severity of skin symptoms, prolong the asymptomatic period or increase its frequency, or regress or prevent recurrence of symptoms. The dose effective for prevention can be determined using a method known to those skilled in the art, for example, by evaluating the activity of an acne strain-selective antibacterial agent according to an embodiment of the present invention in a volunteer subject, in an animal model system allowing prediction of the efficacy in humans, or *in vitro.*

There is no particular restriction on the form of the composition, insofar as it is a form applicable to the skin. Possible examples thereof include a solution system, a solubilized system, an emulsion system, a powdered system, a gel system, an ointment system, a cream, a mist, an aerosol, a water-oil two-phase system, and a water-oil-powder three-phase system. When the form of the composition is a solution system, its pH is larger than 3, for example, pH 3 to 12 depending on the formulation form, and from the viewpoint of skin irritation, for example, pH 3.5 to 8, preferably pH 4 to 7, and more preferably pH 4.5 to 7, or alternatively from the viewpoint of cleaning performance, for example, pH 6 to 12, preferably pH 7 to 12, and more preferably pH 8 to 11.

In an example, the composition is a medicinal composition and is a pharmaceutical or a quasi-drug. In this case, the carrier acceptable for skin application is a pharmaceutically acceptable carrier. In this example, the composition is, for example, an external preparation for skin. The formulation form thereof is not particularly limited, and may be an external solid preparation, an external liquid preparation, a spray, an ointment, a cream, a gel, or a patch. The external preparation for skin may contain a fatty acid or an ester thereof according to the present invention as appropriate corresponding to the formulation form. For example, the external preparation for skin may contain either of them at 0.01 to 5 wt%, preferably 0.05 to 5 wt%, and more preferably 0.1 to 3 wt% with respect to total weight of the preparation.

The external preparation for skin may contain additionally an ingredient other than an antibacterial agent according to the present invention to the extent that the effect of the antibacterial agent is not inhibited or eliminated. There is no particular restriction on such other ingredient, and examples thereof include purified water, lactose, starch, an alcohol, an oily substance, a humectant, a skin aging inhibitor, a whitening agent, a polymer compound, a preservative, a thickener, an emulsifier, a medicinal ingredient, an ultraviolet absorber, a dye stuff, a perfume, an emulsion stabilizer, and a pH adjuster. There is no particular restriction on the method for preparing the external preparation for skin, and it may be produced according to a conventional method. As an example, it can be produced by dissolving a fatty acid or an ester thereof according to the present invention in an appropriate solvent, adding the solution to an aqueous solution, and mixing the same.

In another example, the composition is a cosmetic composition, which may be, but not limited to, a skin care cosmetic, a makeup cosmetic, a body care cosmetic, or a hair care cosmetic.

The "skin care cosmetic" herein includes, but not limited to, a cleaning cosmetic, such as a face wash, and a make-up remover; a skin conditioning cosmetic, such as a cosmetic lotion, a beauty essence, a gel, and a pack (mask); a protective cosmetic, such as a milky lotion, and a cream (moisture cream); a skin-whitening cosmetic. In an embodiment, the skin care cosmetic may be a skin conditioning cosmetic, a protective cosmetic, and a skin-whitening cosmetic.

A "makeup cosmetic" herein includes, but not limited to, a foundation (powder foundation, cake foundation, dual foundation, stick foundation, cream foundation, and liquid foundation), a base makeup cosmetic such as a foundation primer; and a point makeup cosmetic, such as an eye makeup, and a cheek blusher.

A "body care cosmetic" herein may be, but not limited to, soap, a body shampoo, a body lotion, a body cream, a slimming cosmetic, a hand cream, and a bath preparation.

A "hair care cosmetic" herein may be, but not limited to, a hair rinse or a shampoo.

A "clothing item" herein may be, but not limited to, an underwear or a mask (particularly hygiene mask).

The composition may be, but not limited to, in the form of lotion, cream, gel, mousse, stick, spray, tablet, ointment, fiber, or sheet.

The composition may blend appropriately according to need one or more kinds of ingredients commonly used in cosmetic compositions, such as an aqueous humectant; an oily ingredient; an effect-efficacy ingredient, such as a skin-whitening ingredient, a UV protection ingredient, a rough skin ameliorating ingredient, an astringent ingredient, and a horny layer softening ingredient; a quality retaining agent; a surfactant; a coloring agent; a plant extract; and miscellaneous ingredients (such as a saccharide, an amino acid, an organic amine, and a vitamin); to the extent that the acne strain-selective antibacterial activity of the fatty acid or an ester thereof according to the present invention is not inhibited or eliminated.

The "aqueous humectant" refers herein to an ingredient having a high affinity for water to be added for keeping the moisture content contained in the horny layer of the skin, and an aqueous humectant used for a cosmetic use may be used without any particular limitation. Examples of an aqueous humectant that may be used in a cosmetic composition according to the present invention include, but not limited to, 1,2-hexanediol, glycerol, 1,3-butylene glycol, dipropylene glycol, PEGs (such as PEG-75, and PEG-150), propylene glycol, hyaluronic acid, an amino acid, collagen, dextran, a saccharide, and betaine.

An "oily ingredient" means herein an ingredient as a base ingredient for constituting the basis of a cosmetic composition, which is an ingredient to be added having a high hydrophobicity for preventing the moisture contained in the horny layer of the skin from evaporating outward so as to maintain the moisture content, and an oily ingredient used for a cosmetic use (provided the fatty acid or an ester thereof according to the present invention is excluded) may be used without any particular restriction. Examples of an oily ingredient usable in the cosmetic composition according to the present invention include, but not limited to, an oil, such as olive oil, camellia oil, macadamia nut oil, castor oil, and a hydrogenated coconut oil; a wax, such as carnauba wax, jojoba oil, beeswax, and lanolin; a hydrocarbon, such as liquid paraffin, paraffin, petrolatum, ceresin, and squalane; a higher alcohol, such as cetyl alcohol, stearyl alcohol, and 2-octyldodecanol; and a silicone oil, such as methyl polysiloxane, and methylphenyl polysiloxane.

A "whitening ingredient" refers herein to an ingredient that can suppress the synthesis of a melanin pigment related to skin color, and a whitening ingredient used for a cosmetic use may be used without particular limitation. A "UV protection ingredient" refers herein to an ingredient that can protect the skin by absorbing or scattering ultraviolet A rays and ultraviolet ray B rays which may adversely affect the skin, for which a UV protection ingredient used for a cosmetic use may be used without any particular limitation. A "rough skin ameliorating ingredient" refers herein to an ingredient having an activity of preventing or suppressing inflammation of the skin, for which a rough skin ameliorating ingredient used for a cosmetic use may be used without any particular limitation. An "astringent ingredient" refers herein to an ingredient capable of having an effect of astringing the skin, for which an astringent ingredient used for a cosmetic use may be used without any particular limitation. A "horny layer softening ingredient" refers herein to an ingredient capable of having an action of softening the horny layer of the skin, for which a horny layer softening ingredient used for a cosmetic use may be used without any particular limitation.

A "quality retaining agent" refers herein to an ingredient for retaining the stability or safety of the product for a long period of time, and examples thereof include, but not limited to, a thickener, a sequestering agent, a pH adjuster, and a preservative. There is no particular restriction on a "thickener" that can be used in a cosmetic composition according to the present invention, insofar as it is a thickener that can be used for a cosmetic use, and examples thereof include a natural polymer, such as guar gum, carrageenan, gum arabic, tragacanth gum, pectin, xanthan gum, dextran, hyaluronic acid, and collagen; a semisynthetic polymer, such as methyl cellulose, hydroxyethyl cellulose, hydroxypropyl cellulose, carboxymethylcellulose, carboxymethyl starch, an alginate, and another polysaccharide derivative; a synthetic polymer, such as poly(vinyl alcohol), polyvinylpyrrolidone, carboxyvinyl polymer, poly(ethylene oxide), an acrylic acid/alkyl methacrylate copolymer; an inorganic polymer, such as bentonite, and finely pulverized silicon oxide; and a mixture thereof.

There is no particular restriction on a "sequestering agent" that can be used in a cosmetic composition according to the present invention, insofar as it is a sequestering agent used for a cosmetic use, and examples thereof include an EDTA salt (such as EDTA-2Na, and EDTA-3Na), etidronic acid, sodium gluconate, sodium metaphosphate, and a mixture thereof. There is no particular restriction on a "pH adjuster" that can be used in a cosmetic composition according to the present invention, insofar as it is a pH adjuster used for a cosmetic use, and examples thereof include citric acid, malic acid, sodium hydroxide, potassium hydroxide, and a mixture thereof. There is no particular restriction on a "preservative" that can be used in a cosmetic composition according to the present invention, insofar as it is a preservative used for a cosmetic use, and examples thereof include chlorphenesin, potassium sorbate, sodium dehydroacetate, a paraben (*e.g.* methylparaben, and propylparaben), phenoxyethanol, methylchloroisothiazolinone, and a mixture thereof.

There is no particular restriction on a "surfactant" hereunder, insofar as it is a surfactant which is an ingredient having a hydrophilic moiety (hydrophilic group) and a lipophilic moiety (lipophilic group) in one molecule, and is used for a cosmetic use, and examples thereof include an anionic surfactant, a cationic surfactant, a nonionic surfactant, an amphoteric surfactant, and a mixture thereof. In an embodiment, a cosmetic composition according to the present invention comprises a cationic surfactant, a nonionic surfactant, an amphoteric surfactant, or a mixture thereof, preferably at least a nonionic surfactant, and more preferably only a nonionic surfactant as a surfactant. There is no particular restriction on a "coloring agent" hereunder, insofar as it is a coloring agent used for a cosmetic use, and examples thereof include titanium oxide, silica, talc, manganese violet, and a mixture thereof. A "plant extract" may be used hereunder without any particular restriction, insofar as it is a plant extract used for a cosmetic use.

The cosmetic composition and the clothing can be produced respectively according to a known production method by adding a fatty acid or an ester thereof according to the present invention, or an acne strain-selective antibacterial agent according to an embodiment of the present invention, or by substituting the same for part of known raw materials. When the cosmetic composition contains a surfactant, although it is not essential, the content thereof with respect to the total weight of the composition is 2 wt% or less, preferably 1 wt% or less, and more preferably 0.5 wt% or less. More preferably, the cosmetic composition is substantially free from a surfactant, or free from it. The expression of "substantially free from a surfactant" means herein that the content with respect to the total weight of the composition is less than 0.1 wt%, and it in an embodiment less than 0.05 wt%, and in another embodiment less than 0.01 wt%.

Many of the cosmetic compositions according to the present invention may be produced by, but not limited to, dispersing an acne strain-selective antibacterial agent according to an embodiment of the present invention, water, and an oily ingredient (such as a liquid oil, a solid fat, a wax, a hydrocarbon, a higher alcohol, an ester oil, or a silicone oil, etc.) using a thickener.

As one example, for example a cosmetic lotion is prepared by blending a fatty acid or an ester thereof according to the present invention together with known ingredients to be blended in a conventional cosmetic lotion according to a conventional method. Examples of such known ingredients include, but not limited to, an aqueous humectant, a preservative, a perfume, an oily ingredient, a thickener, an alcohol, a surfactant (solubilizing agent), and a plant extract. These optional ingredients can be blended singly or in a mixture of two or more kinds thereof corresponding to the application of the cosmetic lotion. For example, a cosmetic lotion may be produced by dissolving a fatty acid or an ester thereof according to the present invention and a surfactant (for example 0.1 to 0.3 wt%) in an alcohol (for example ethanol), and mixing the solution with an aqueous solution containing an aqueous humectant and a preservative.

As another example, for example a gel cosmetic is prepared by blending a fatty acid or an ester thereof according to the present invention together with known ingredients to be blended in a conventional gel cosmetic according to a conventional method. Examples of such known ingredients include, but not limited to, an aqueous humectant, a preservative, a perfume, an oily ingredient, a thickener, an alcohol, a surfactant, an ultraviolet absorber, and a sequestering agent. These optional ingredients can be blended singly or in a mixture of two or more kinds thereof corresponding to the application of the gel cosmetic. For example, a gel cosmetic may be produced by dissolving together a fatty acid or an ester thereof according to the present invention, an alcohol (for example ethanol), and an oily ingredient, and mixing the solution with an aqueous solution containing a thickener, an aqueous humectant, and a sequestering agent. Alternatively, a gel cosmetic may be produced by adding and mixing a fatty acid or an ester thereof according to the present invention, and a surfactant (for example, 0.1 to 1.0 wt%) to an aqueous solution containing a thickener and an aqueous humectant.

As another example, for example a cream cosmetic is prepared by blending a fatty acid or an ester thereof according to the present invention together with known ingredients to be blended in a conventional cream cosmetic according to a conventional method. Examples of such known ingredients include, but not limited to, an aqueous humectant, a preservative, a perfume, an oily ingredient, a surfactant, and an ultraviolet absorber. These optional ingredients can be blended singly or in a mixture of two or more kinds thereof corresponding to the application of the cream cosmetic. For example, a cream cosmetic may be produced by heating and mixing together a fatty acid or an ester thereof according to the present invention, an oily ingredient, and a surfactant (for example, 0.5 wt% to 1.5 wt%) for dissolution, and mixing the solution with an aqueous solution containing an aqueous humectant, followed by cooling.

As an example, for example soap is prepared by blending a fatty acid or an ester thereof according to the present invention with a known ingredient blended into a conventional soap according to a conventional method. Examples of such a known ingredient include, but not limited to, a humectant, a preservative, a perfume, an oily ingredient, a lubricant, a water soluble polymer, a soap base, a chelating agent, a surfactant, a higher alcohol, an antioxidant, a sequestering agent, and an anti-inflammatory agent. These known ingredients may be blended singly, or in a mixture of two or more kinds corresponding to the application or the formulation form of the soap.

As another example, for example, a bath preparation may be prepared according to a conventional method by using an acne strain-selective antibacterial agent according to an embodiment of the present invention together with a known ingredient blended in a conventional bath agent. Examples of such a known ingredient include, but not limited to, a carbonate, an organic acid, an inorganic pigment, a humectant, a preservative, a perfume, an inorganic salt, an oily ingredient, a lubricant, a water soluble polymer, a coloring agent, and a plant extract. These known ingredients may be blended singly, or in a mixture of two or more kinds corresponding to the formulation form or the application of a bath preparation.

A composition according to an embodiment of the present invention comprises the antibacterial agent at a content of from 0.001 to 10 wt% with respect to the total weight of the composition, for example from 0.01 to 5 wt%, or from 0.05 to 2 wt%. In an embodiment, the composition contains a pharmaceutically acceptable carrier at a content of from 50 to 99 wt% with respect to the total weight of the composition, for example from 80 to 95 wt%.

As the third aspect, the present invention provides use of at least one fatty acid selected from the group consisting of saturated fatty acids having 14 to 20 carbon atoms (C14:0 to C20:0) and a monounsaturated fatty acid having 18 carbon atoms (C18:1) in a free form or a salt form, or an ester thereof, for producing an acne strain-selective antibacterial agent or a skin-care composition. The features described above with respect to an acne strain-selective antibacterial agent, and a skin-care composition, as well as a fatty acid or an ester thereof apply also to the antibacterial agent or the skin-care composition, and the fatty acid or an ester thereof of this aspect.

As the fourth aspect, the present invention provides a treating method and a preventing method of acne including application of a skin-care composition according to an embodiment of the present invention to the skin of a subject in need thereof (for example, human). The features with respect to a skin-care composition, as well as a fatty acid or an ester thereof described above apply also to a composition, as well as a fatty acid or an ester thereof of the present aspect.

Examples will be described below as exemplary embodiments of the present invention, provided that they do not limit in any way the invention defined in the Claims attached hereto.

### Examples

### <Example 1>

### Acne strain-selective antibacterial activity

The antibacterial activity of a test substance was determined based on the minimum inhibitory concentration (MIC). An MIC was measured according to the broth microdilution method which is the standard method of Japanese Society of Chemotherapy.

### (1) Preparation of strain suspension

One-loopful of each acne bacterium strain listed in Table 1 was scraped off and inoculated into 5 mL of a GAM broth slant agar medium (pH 7.0, Nissui Pharmaceutical Co., Ltd.).

**[Table 1]**

| Acne bacterium strain | Ribotype | Features |
|---|---|---|
| HL053PA1 strain (HM-522^{†}) | RT4 | Dominant in acne patients |
| HL043PA1 strain (HM-513^{†}) | RT5 | Dominant in acne patients |
| HL110PA3 strain (HM-554^{†}) | RT6 | Dominant in healthy subjects |

| | | |
|---|---|---|
| †: Number in the catalog of BEI Resources (Manassas, VA, USA) | | |

The inoculated acne bacterium strain was precultured at 37°C in a nitrogen atmosphere (anaerobic condition) for 48 hours. The precultured microbial cells were diluted in a modified GAM broth liquid medium (pH 6.0) to a concentration of 5.0 × 10⁴ cfu/mL (viable cell count per OD₆₆₀ = 1 is 2.0 × 10⁹ cfu/mL), to prepare strain suspensions.

### (2) Preparation of test substance

Each fatty acid (Tokyo Chemical Industry Co., Ltd.) listed in Table 2 was dissolved in dimethylsulfoxide (DMSO) as a solvent such that the concentration became 10,000 µg/mL, and used as a test substance.

### (3) MIC measurement

Each of strain suspensions (5.0 × 10⁴ cfu/mL) was aliquoted in a 96-well round bottom microplate, each of the test substance (fatty acid concentration 10,000 µg/mL) was added at a 1/10 volume to the aliquot and mixed to prepare measurement samples (fatty acid concentration: 1,000 µg/mL). Measurement samples in 2-fold dilution series were prepared using the strain suspension (fatty acid concentration from 0.48 to 1,000 µg/mL). Using a sample containing only the solvent (DMSO) as a control, it was confirmed that the influence of the solvent on the growth of each strain could be neglected.

The measurement samples were incubated under an anaerobic condition at 37°C for 2 days. The presence or absence of a precipitation of test microbial cells after the incubation was examined and the minimum concentration at which the precipitation of microbial cells was recognized was regarded as the MIC (µg/mL) of the test substance against the acne bacterium strain. The obtained MIC is shown in Table 2.

**[Table 2]**

| Test substance | MIC (µg/mL) against acne bacterium strain | | | Strain-selective antibacterial activity | Rating |
|---|---|---|---|---|---|
| | HL053PA1 strain (RT4) | HL043PA1 strain (RT5) | HL110PA3 strain (RT6) | | |
| Caproic acid (C6:0) | 62.5 | 62.5 | 62.5 | 1.0 | |
| Heptanoic acid (C7:0) | 125 | 125 | 125 | 1.0 | - |
| Caprylic acid (C8:0) | 250 | 250 | 125 | 0.50 | - |
| Nonanoic acid (C9:0) | 500 | 250 | 250 | 0.75 | - |
| Capric acid (C10:0) | 500 | 500 | 250 | 0.50 | - |
| Undecanoic acid (C11:0) | 15.6 | 15.6 | 15.6 | 1.0 | - |
| Lauric acid (C12:0) | 15.6 | 31.3 | 15.6 | 0.75 | - |
| Tridecanoic acid (C13:0) | 15.6 | 31.3 | 15.6 | 0.75 | - |
| Myristic acid (C14:0) | 15.6 | 15.6 | 250 | 16 | ++ |
| Pentadecanoic acid (C15:0) | 1.95 | 1.95 | 250 | 130 | ++++ |
| Palmitic acid (C16:0) | 7.81 | 7.81 | > 1000 | > 130 | ++++ |
| Heptadecanoic acid (C17:0) | 7.81 | 7.81 | > 1000 | > 130 | ++++ |
| Stearic acid (C18:0) | > 1000 | > 1000 | > 1000 | ND^{†} | NE^{‡} |
| Palmitoleic acid (C16:1) | 31.3 | 7.81 | 15.6 | 1.2 | +/- |
| Oleic acid (C18:1) | 500 | 62.5 | > 1000 | > 9.0 | ++ |

| | | | | | |
|---|---|---|---|---|---|
| †: Not Determined ‡: Not Evaluable (The same also applies to the following Examples and Comparative Examples.) | | | | | |

The strain-selective antibacterial activity of each test substance (Table 2, right-most column) was evaluated based on the following rating criteria (Table 3). When the strain-selective antibacterial activity is indicated by a numerical range, the rating was based on the minimum value thereof.

**[Table 3]**

| (Rating criteria) | | |
|---|---|---|
| Strain-selective antibacterial activity | Rating | |
| More than 60 | ++++ | : Excellent strain-selective antibacterial activity |
| More than 20 and not more than 60 | +++ | : Very good strain-selective antibacterial activity |
| More than 6 and not more than 20 | ++ | : Good strain-selective antibacterial activity |
| More than 2 and not more than 6 | + | : Practically acceptable strain-selective antibacterial activity |
| More than 1 and not more than 2 | +/- | : Growth of dominant strain in acne patients can be suppressed selectively, |
| Not more than 1 | - | : Growth of a dominant strain in acne patients can not necessarily be suppressed selectively. |
| ND | NE | : Not Evaluable |

Saturated fatty acids (C14:0 to C17:0) and monounsaturated fatty acids (C16:1 and C18:1) exhibited a strain-selective antibacterial activity greater than 1 (>1.0) (Table 2). This indicates that these fatty acids selectively suppress the growth of dominant strains in acne patients (RT4 strain and/or RT5 strain) rather than the growth of a dominant strain in healthy subjects (RT6 strain), and as a result the proportion of the dominant strain in healthy subjects (RT6 strain) in the total acne bacteria can be increased. Since the strain-selective antibacterial activity of palmitoleic acid (C16:1) is as low as 1.2 (Table 2), it is suggested that this acid is not practical as a strain-selective antibacterial agent which suppresses selectively the growth of a dominant strain in acne patients compared to the growth of a dominant strain in healthy subjects.

The saturated fatty acids C6:0 to C13:0 exhibited a strain-selective antibacterial activity of 1 or less (Table 2). This suggests that these fatty acids nonselectively suppress the growth of a dominant strain in healthy subjects (RT6 strain) and the growth of dominant strains in acne patients (RT4 strain and RT5 strain), or selectively suppress the growth of a dominant strain in healthy subjects (RT6 strain) rather than the growth of dominant strains in acne patients (RT4 strain and RT5 strain) (Table 2), and as a result are not able to increase the proportion of the dominant strain in healthy subjects in the total acne bacteria.

The fatty acids (C15:0 to C17:0) among the saturated fatty acids (C14:0 to C17:0) and the monounsaturated fatty acid (C18:1) which exhibited a practical strain-selective antibacterial activity under the measurement conditions of Example 1 exhibited a high strain-selective antibacterial activity (> 128) (Table 2). This suggests that the acid is able to increase effectively the proportion of a dominant strain in healthy subjects in the total acne bacteria over a wide concentration range.

### <Comparative Example 1>

### Strain-selective antibacterial activity of control antibacterial agent

Except that 39 kinds of control antibacterial agents commonly used as an antibacterial agent were used as test substances, and solvents suitable for the respective antibacterial agents were used, the respective strain-selective antibacterial activities were evaluated by measuring MIC by substantially the same method as in Example 1. Among the 39 measured control antibacterial agents, 21 were listed below, and the MIC (µg/mL) and ratings for 5 kinds of control antibacterial agents with respect to an acne bacterium strain are shown in Table 4. Control antibacterial agents
(1) salicylic acid, (2) glycerol fatty acid ester, (3) sodium benzoate, (4) methyl paraoxybenzoate, (5) ethyl paraoxybenzoate, (6) propyl paraoxybenzoate, (7) butyl paraoxybenzoate, (8) glycerol mono(2-ethylhexyl) ether, (9) phenoxyethanol, (10) sodium lauroyl sarcosinate, (11) cetylpyridinium chloride, (12) *N-*capryloyl acylglycine, (13) *N*-coconut oil fatty acid acyl-L-arginine ethyl DL-pyrrolidone carboxylate, (14) isopropylmethylphenol, (15) zinc PCA, (16) benzalkonium chloride, (17) triclosan, (18) zinc gluconate, (19) hinokitiol, (20) *p*-dimethylaminostyryl heptyl methyl thiazolium iodide, and (21) ethanol.

**[Table 4]**

| Test substance | MIC (µg/mL) against acne bacterium strain | | | Strain-selective antibacterial activity | Rating |
|---|---|---|---|---|---|
| | HL053PA1 strain (RT4) | HL043PA1 strain (RT5) | HL110PA3 strain (RT6) | | |
| Cetyl pyridinium chloride | 15.6 | 7.81 | 3.9 | 0.37 | - |
| Isopropyl methylphenol | 125 | 125 | 62.5 | 0.50 | - |
| Triclosan | 0.975 | 1.95 | 3.9 | 3.0 | + |
| Hinokitiol | 31.3 | 31.3 | 31.3 | 1.0 | - |
| Ethanol | > 1000 | 500 | 500 | < 0.75 | - |

All the antibacterial agents other than triclosan exhibited a strain-selective antibacterial activity of 1 or less, which suggests that they are not able to increase the proportion of a dominant strain in healthy subjects in the total acne bacteria.

The strain-selective antibacterial activity of triclosan is 3.0 (Table 4), which suggests that triclosan is able to suppress selectively the growth of dominant strains in acne patients (RT4 strain and RT5 strain) so as to increase the proportion of a dominant strain in healthy subjects (RT6 strain) in the total acne bacterium strains. In this regard, the fatty acids which were demonstrated to exhibit a practical strain-selective antibacterial activity in Example 1 exhibited a strain-selective antibacterial activity beyond 9 (Table 2).

### <Comparative Example 2>

### Strain-selective antibacterial activity of control antibiotic

Except that an existing antibiotic widely used for treatment of acne was used as a test substance, a solvent suitable for each antibiotic was used, and a different concentration range of the target substance was used, the strain-selective antibacterial activities were evaluated by measuring MIC (ng/mL) of the respective test substances against the acne bacterium strain by substantially the same method as in Example 1 (Table 5).

**[Table 5]**

| Test substance | MIC (ng/mL) against acne bacterium strain | | | Strain-selective antibacterial activity | Rating |
|---|---|---|---|---|---|
| | HL053PA1 strain (RT4) | HL043PA1 strain (RT5) | HL110PA3 strain (RT6) | | |
| Doxycycline | 980 | 2,000 | 0.48 | 3.6 × 10⁻⁴ | - |
| Minocycline | 240 | 490 | LOD^{†} | < 1.5 × 10⁻³ | - |
| Clindamycin | 31,000 | 63,000 | 31 | 7.5 × 10⁻⁴ | - |
| Roxithromycin | 500,000 | 1,000,000 | 3.8 | 5.7 × 10⁻⁶ | - |
| Nadifloxacin | 31 | 31 | LOD^{†} | < 1.5 × 10⁻² | - |

| | | | | | |
|---|---|---|---|---|---|
| t: Not more than the limit of detection (LOD). In the case of Comparative Example 1, it means that at least MIC was < 0.48 ng/mL. ‡: Calculated using 0.48 ng/mL as the value of MIC of the RT6 strain. | | | | | |

Unexpectedly, the antibiotics for treating acne used in Comparative Example 2 suppressed selectively the growth of the dominant strain in healthy subjects (RT6 strain), and it was suggested as a result that the proportion of the dominant strains in acne patients (RT4 strain and RT5 strain) in the total acne bacteria would be increased.

### <Example 2>

### Strain-selective antibacterial activity at various pH values

To evaluate the strain-selective antibacterial activity (Tables 6 to 9), the MIC (µg/mL) of each test substance against the acne bacterium strain was measured substantially in the same manner as in Example 1 except that each of the saturated fatty acids (C14:0 to C17:0) having exhibited a high strain-selective antibacterial activity, and the saturated fatty acid (C18:0) having been resulted in ND in Example 1 was used as a test substance, and that the pH of the modified GAM broth liquid medium was changed from 6.0 to 5.0, 5.5, 6.5, and 7.0.

**[Table 6]**

| MIC^{†} (µg/mL) of test substance at pH 5.0 | | | | | |
|---|---|---|---|---|---|
| Test substance | MIC (µg/mL) against acne bacterium strain | | | Strain-selective antibacterial activity | Rating |
| | HL053PA1 strain (RT4) | HL043PA1 strain (RT5) | HL110PA3 strain (RT6) | | |
| Myristic acid (C14:0) | 15.6 | 31.3 | > 1000 | > 48 | +++ |
| Pentadecanoic acid (C15:0) | 7.81 | 15.6 | > 1000 | > 96 | ++++ |
| Palmitic acid (C16:0) | > 1000 | > 1000 | > 1000 | ND | NE |
| Heptadecanoic acid (C17:0) | > 1000 | > 1000 | > 1000 | ND | NE |
| Stearic acid (C18:0) | > 1000 | > 1000 | > 1000 | ND | NE |

| | | | | | |
|---|---|---|---|---|---|
| †: Considering the growth rate of a strain, the MICs of RT4, RT5 and RT6 were estimated from the growth on day 3, day 3, and day 4. | | | | | |

**[Table 7]**

| MIC^{†} (µg/mL) of test substance at pH 5.5 | | | | | |
|---|---|---|---|---|---|
| Test substance | MIC (µg/mL) against acne bacterium strain | | | Strain-selective antibacterial activity | Rating |
| | HL053PA1 strain (RT4) | HL043PA1 strain (RT5) | HL110PA3 strain (RT6) | | |
| Myristic acid (C14:0) | 15.6 | 15.6 | > 1000 | > 64 | ++++ |
| Pentadecanoic acid (C15:0) | 3.9 | 3.9 | > 1000 | > 260 | ++++ |
| Palmitic acid (C16:0) | 62.5 | 62.5 | > 1000 | > 16 | ++ |
| Heptadecanoic acid (C17:0) | 15.6 | 15.δ | > 1000 | > 64 | ++++ |
| Stearic acid (C18:0) | > 1000 | > 1000 | > 1000 | ND | NE |

| | | | | | |
|---|---|---|---|---|---|
| †: Considering the growth rate of a strain, the MIC of RT6 was estimated from the growth on day 3. | | | | | |

**[Table 8]**

| MIC (µg/mL) of test substance at pH 6.5 | | | | | |
|---|---|---|---|---|---|
| Test substance | MIC (µg/mL) against acne bacterium strain | | | Strain-selective antibacterial activity | Rating |
| | HL053PA1 strain (RT4) | HL043PA1 strain (RT5) | HL110PA3 strain (RT6) | | |
| Myristic acid (C14:0) | 125 | 31.3 | 250 | > 5.0 | + |
| Pentadecanoic acid (C15:0) | 3.9 | 1.95 | 250 | > 96 | ++++ |
| Palmitic acid (C16:0) | 125 | 3.9 | > 1000 | > 130 | ++++ |
| Heptadecanoic acid (C17:0) | 31.3 | 3.9 | > 1000 | > 140 | ++++ |
| Stearic acid (C18:0) | > 1000 | > 1000 | > 1000 | ND | NE |

**[Table 9]**

| MIC (µg/mL) of test substance at pH 7.0 | | | | | |
|---|---|---|---|---|---|
| Test substance | MIC (µg/mL) against acne bacterium strain | | | Strain-selective antibacterial activity | Rating |
| | HL053PA1 strain (RT4) | HL043PA1 strain (RT5) | HL110PA3 strain (RT6) | | |
| Myristic acid (C14:0) | 250 | 250 | 500 | 2.0 | +/- |
| Pentadecanoic acid (C15:0) | 3.9 | 3.9 | 1000 | 260 | ++++ |
| Palmitic acid (C16:0) | 62.5 | 15.6 | 1000 | 40 | +++ |
| Heptadecanoic acid (C17:0) | 62.5 | 7.8 | 1000 | 72 | ++++ |
| Stearic acid (C18:0) | 62.5 | 31.3 | 1000 | 24 | +++ |

The strain-selective antibacterial activities and the ratings of myristic acid, pentadecanoic acid, palmitic acid, heptadecanoic acid, stearic acid, and oleic acid at each pH exhibited in Examples 1 and 2 are summarized in Table 10.

**[Table 10]**

| Test substance | Strain-selective antibacterial activity at each pH (Rating) | | | | |
|---|---|---|---|---|---|
| | pH5 | pH5.5 | pH6 | pH6.5 | pH7 |
| Myristic acid (C14:0) | > 48 (+++) | > 64 (++++) | 16 (++) | > 5.0 (+) | 2.0 (+/-) |
| Pentadecanoic acid (C15:0) | > 96 (++++) | > 260 (++++) | 130 (++++) | > 96 (++++) | 260 (++++) |
| Palmitic acid (C16:0) | ND (NE) | > 16 (++) | > 130 (++++) | > 130 (++++) | 40 (+++) |
| Heptadecanoic acid (C17:0) | ND (NE) | > 64 (++++) | > 130 (++++) | > 140 (++++) | 72 (++++) |
| Stearic acid (C18:0) | ND (NE) | ND (NE) | ND (NE) | ND (NE) | 24 (+++) |
| Oleic acid (C18:1) | NCW^{†} | NCW | > 9 (++) | NCW | NCW |

| | | | | | |
|---|---|---|---|---|---|
| t: Not Complied With (Not tested) | | | | | |

There was a tendency that the MIC of a saturated fatty acid having a relatively long chain against a dominant strain in acne patients (MIC_{RT4} and MIC_{RT5}) decreased as the pH changed from a weakly acidic range to near the neutral range (Tables 6 to 9). For example, the MIC_{RT4} and MIC_{RT5} of the saturated fatty acids (C16:0 and C17:0) were both > 1000 µg/mL at pH 5.0 (Table 6), however they decreased to 3.9 to 130 µg/mL in a range from pH 5.5 to 7.0 (Tables 7 to 9). The strain-selective antibacterial activities of the saturated fatty acids were as high as 16 to 140 (Tables 7 to 9). The MIC of stearic acid (C18:0) which was the saturated fatty acid having the longest chain among the test substances could be determined at pH 7.0 under the measurement conditions of Example 2, and its strain-selective antibacterial activity was as high as 24 (Table 9). Pentadecanoic acid (C15:0) maintained a high strain-selective antibacterial activity over the entire pH range tested (pH 5 to 7) (Table 10). Heptadecanoic acid (C17:0) maintained a high strain-selective antibacterial activity between pH 5.5 and pH 7 in the tested pH range. Further, pentadecanoic acid also maintained a high strain-selective antibacterial activity between pH 5.5 and pH 7.

Since the fatty acids C14:0 to C20:0 and C18:1 could suppress selectively the growth of the dominant strain in acne patients compared to the growth of the dominant strain in healthy subjects as obvious from Examples 1 and 2, it was consequently suggested that they could increase the proportion of the dominant strain in healthy subjects in the total acne bacteria.

### <Example 3>

### Strain-selective antibacterial activity of monoacylglycerol (MAG)

### (1) Preparation of monoacylglycerol

A monoacylglycerol of a fatty acid (C14 to C18) was prepared according to the method described in Praphan Pinsirodom, et al., J. Am. Oil Chem. Soc., 81, 543-547 (2004). Specifically, 1,000 mg of pentadecanoic acid (C15:0), 1,900 mg of glycerol (5 fold molar amount of the fatty acid), 116 mg of lipase derived from *Penicillium camemberti* (Lipase G, Amano Enzyme Inc.) were added into 0.058 mL of water, and the mixture was reacted at 30°C for 2 days with stirring. After the reaction, 15 mL of chloroform/methanol (2:1, v/v) and 5 mL of water were added, and the mixture was stirred, and then centrifuged, and the solvent layer was recovered. The recovered solvent layer was concentrated by distilling off the solvent with an evaporator. The obtained concentrate was loaded on a silica gel chromatographic column, such that contaminants were eluted using hexane/ethyl acetate (80:20, v/v). Subsequently, a monoacylglycerol was eluted with methanol to obtain 0.41 g of monopentadecanoin. Monoacylglycerols of other fatty acids (C14:0, C16:0, C17:0 and C18:0) were prepared in the same manner.

### (2) MIC measurement of monoacylglycerol

Using each of the prepared monoacylglycerols of fatty acids as a test substance, the MIC (µg/mL) of each test substance against an acne bacterium strain was measured in substantially the same manner as in Example 1 (Table 11).

**[Table 11]**

| Test substance | MIC (µg/mL) against acne bacterium strain | | | Strain-selective antibacterial activity | Rating |
|---|---|---|---|---|---|
| | HL053PA1 strain (RT4) | HL043PA1 strain (RT5) | HL110PA3 strain (RT6) | | |
| Monomyristin C14:0MAG | 500 | 500 | > 1000 | > 2.0 | + |
| Monopentadecanoin C15:0MAG | 15.6 | 7.81 | 62.5 | 6.0 | + |
| Monopalmitin C16:0MAG | 250 | 31.3 | 1000 | 18 | ++ |
| C17:0MAG | 250 | 15.6 | 1000 | 34 | ++++ |
| Monostearin C18:0MAG | > 1000 | > 1000 | > 1000 | ND | NE |

MAGs of saturated fatty acids (C14:0 to C17:0) exhibit a strain-selective antibacterial activity of more than 2 (> 2.0) (Table 11) indicating a good strain-selective antibacterial activity. Under the measurement condition, it was not possible to determine (ND) the acne strain-selective antibacterial activity of the MAG of stearic acid (C18:0).

It was demonstrated that an ester (monoacylglycerol) of a fatty acid according to the present invention suppressed or inhibited selectively the growth of a dominant strain in acne patients compared to a dominant strain in healthy subjects (Example 3). It has been suggested by the study of the present inventors that the expression level of lipase is higher in a dominant strain in acne patients than in a dominant strain in healthy subjects (the data are omitted here). Putting all the results together, it can be deduced that in the vicinity of a dominant strain in acne patients where a relatively large amount of lipase is expressed, a free fatty acid according to the present invention originated from an ester thereof is abundant compared to in the vicinity of a dominant strain in healthy subjects, and consequently, the growth of the dominant strain in acne patients can be suppressed or inhibited selectively compared to the growth of the dominant strain in healthy subjects. The above mechanism is not limited to a monoacylglycerol, and may also apply to another fatty acid ester, which can be decomposed by a lipase to give a free fatty acid, such as a diacylglycerol, a triacylglycerol, a cholesterol ester, and a wax ester.

### <Example 4>

### Time course of strain-selective antibacterial activity

To each strain suspension (pH 6.0, 5.0 × 10⁵ cfu/mL) of the acne bacterium strain (HL053PA1 strain (RT4 strain), HL043PA1 strain (RT5 strain), and HL110PA3 strain (RT6 strain)) prepared in the same manner as in Example 1, pentadecanoic acid (C15:0) was added to mix together (final concentration of a fatty acid of 15 µg/mL, and final concentration of solvent DMSO of 0.5%), and each the mixture was statically cultured at 37°C in a nitrogen atmosphere (anaerobic condition). Using a sample to which only DMSO was added at 0.5% (final concentration) as a control, it was confirmed that the influence of the solvent on the growth of each strain could be neglected.

The viable cell count in the culture solution was measured at 0, 2, 8, 18, 30 and 44 hours after the start of culturing. A viable cell count (cfu/mL) was calculated from the number of emerged colonies after culturing part of the culture solution on a GAM broth agar plate under anaerobic conditions for 3 days (Figure 1) .

The growth of the HL053PA1 strain (RT4 strain) which was a dominant strain in acne patients was bacteriostatically suppressed in the presence of the fatty acid C15:0 (Figure 1a). The growth of the HL043PA1 strain (RT5 strain) which was a dominant strain in acne patients was suppressed first bacteriostatically and then bactericidally after 10 hours from the start of culturing in the presence of pentadecanoic acid (C15:0) (Figure 1b). It was demonstrated that the growth of the HL110PA3 strain (RT6 strain) which was a dominant strain in healthy subjects was not suppressed by the presence of pentadecanoic acid (C15:0) (Figure 1c).

With an acne strain-selective antibacterial agent according to the present invention, a skin indigenous microbial flora including acne bacteria in which the proportion of a dominant strain in healthy subjects is enhanced can be produced. The presence of such a skin indigenous microbial flora in which the proportion of a bacterium strain having a favorable action has been enhanced is preferable, because the growth of bacteria that can deteriorate the skin environment may be suppressed. For example, it is known that acne bacteria can suppress the growth of *Staphylococcus aureus* which is a causative bacterium of adult acne. Owing to the acne strain-selective antibacterial agent according to the present invention, it can be expected that the growth of *Staphylococcus aureus* may be suppressed by acne bacteria in the indigenous microbial flora of the skin where the proportion of the dominant strain in healthy subjects is enhanced, so as to prevent or treat adult acne.

### <Example 5>

### Strain-selective antibacterial activity in coculture of acne bacterium strains

### (1) Preparation of coculturing strain suspension

Acne bacteria strains (HL053PA1 strain (RT4 strain), HL043PA1 strain (RT5 strain), and HL110PA3 strain (RT6 strain)) were respectively precultured in the same manner as in Example 1.

The HL053PA1 strain (RT4 strain) and the HL110PA3 strain (RT6 strain) were suspended in a modified GAM broth liquid medium (pH 6.0) respectively at a concentration of 5.0 × 10⁴ cfu/mL. Similarly, the HL043PA1 strain (RT5 strain) and the HL110PA3 strain (RT6 strain) were suspended respectively at a concentration of 5.0 × 10⁴ cfu/mL. The suspensions were respectively designated as coculturing strain suspensions-1, and -2.

### (2) Coculture test

Pentadecanoic acid (C15:0) was added to the prepared coculturing strain suspensions-1 and -2 to prepare test samples (final concentration 15 and 0.49 µg/mL). Using a sample to which only 0.5% of DMSO was added as a control, it was confirmed that the influence of the solvent on the growth of each bacterium strain could be neglected.

The test sample was statically cultured under anaerobic conditions at 37°C for 2 days. After the culture, part of the culture solution was cultured under anaerobic conditions on a GAM broth agar plate (pH 7) for 3 days. The bacterium strains were identified based on the morphology (particularly size) of the emerged colonies, and the viable cell count (cfu/mL) of each bacterium strain was measured. From the measurement results, the proportion of each bacterium strain in the entire microbial cells was calculated (Figure 2).

When co-culturing was performed in a modified GAM broth liquid medium (pH 6.0) in the absence of the fatty acid (C15:0), the proportion of the dominant strain in acne patients (HL053PA1 strain (RT4 strain), or HL043PA1 strain (RT5 strain)) became higher than the proportion of the dominant strain in healthy subjects (HL110PA3 strain (RT6 strain) (Figure 2 (-)). This may reflect a fact that the dominant strain in acne patients has a growth rate higher than the dominant strain in healthy subjects in a weakly acidic environment (pH 6.0).

However, in the presence of the fatty acids (C15:0, 15.6 µg/mL) under the same weakly acidic condition (modified GAM broth liquid medium, pH 6.0), the proportion of the dominant strain in healthy subjects became extremely high (Figure 2, (-) vs. (+, 15.6)). The growth of the dominant strain in acne patients was almost completely suppressed by the presence of this fatty acid. The concentration of the fatty acid (C15:0) used was higher than MIC_{RT4} (1.95 µg/mL) and MIC_{RT5} (1.95 µg/mL) measured under the same weakly acidic condition (Example 1), and lower than MIC_{RT6} (250 µg/mL) (Table 2).

Even when the concentration of the fatty acid (C15:0) was set at 0.45 µg/mL, which was lower than MIC_{RT4}, MIC_{RT5}, and MIC_{RT6} measured under the same weakly acidic condition (Example 1), the growth of the dominant strains in acne patients (in particular, HL043PA1 strain (RT5 strain)) was suppressed selectively, and the proportion of the dominant strain in healthy subjects in the total acne bacteria increased (Figure 2, (-) *vs.* (+, 0.45)).

These results indicate that in a case where an acne strain-selective antibacterial agent according to the present invention is blended in a composition, or a skin care cosmetic, such as a cosmetic at an effective dose based on its MIC value as a guide, the growth of a dominant strain in acne patients may be selectively suppressed so as to enhance the proportion of a dominant strain in healthy subjects in the total acne bacteria.

### <Example 6>

### Strain-selective antibacterial activity against different acne bacterium strains

The MIC of each of the following test substances was measured substantially in the same manner as in Example 1 (Table 12) except that a suspension of test bacterium strains was prepared using the dominant strain in acne patients (HL005PA1 strain (HM-492^{†}, RT4 strain), HL0565PA1 strain (HM-524^{†}, RT4 strain), HL045PA1 strain (HM-516^{†}, RT4 strain), or HL043PA2 strain (HM-514^{†}, RT5 strain)), or the dominant strain in healthy subjects (HL110PA4 strain (HM-555^{†}, RT6 strain) instead of the dominant strains in acne patients (HL053PA1 strain (RT4 strain) and HL043PA1 strain (RT5 strain)) and the dominant strain in healthy subjects (HL110PA3 strain (RT6 strain) used in Examples 1 to 3.
††: Number in the catalog of BEI Resources (Manassas, VA, USA)

**[Table 12]**

| Test substance | MIC (µg/mL) against acne bacterium strain | | | | | Strain-selective antibacterial activity | Rating |
|---|---|---|---|---|---|---|---|
| | HL005 PA1 strain (RT4) | HL056 PA1 strain (RT4) | HL045 PA1 strain (RT4) | HL043 PA2 strain (RT5) | HL110 PA4 strain (RT6) | | |
| Myristic acid (C14:0) | 31.3 | 125 | 31.3 | 15.6 | 250 | 8.5 | ++ |
| Pentadecanoic acid (C15:0) | 3.9 | 3.9 | 3.9 | 3.9 | 250 | 64 | ++++ |
| Palmitic acid (C16:0) | 62.5 | 500 | 250 | 62.5 | > 1000 | > 9.5 | ++ |
| Heptadecanoic acid (C17:0) | 62.5 | 250 | 125 | 62.5 | > 1000 | > 11 | ++ |

Any of the tested saturated fatty acids (C14:0 to C17:0) suppressed selectively the growth of the dominant strains in acne patients (RT4 strain and RT5 strain) rather than the growth of the dominant strain in healthy subjects (RT6 strain) with respect to the tested bacterium strains (Tables 2 and 12). It has been suggested that an acne strain-selective antibacterial agent according to the present invention is capable of suppressing selectively the growth of the dominant strains in acne patients (RT4 strain and RT5 strain) rather than the growth of the dominant strain in healthy subjects (RT6 strain) without any particular limitation to a specific strain of acne bacteria, and consequently enhancing the proportion of the dominant strain in healthy subjects (RT6 strain) in the entire acne bacteria.

## Claims

1. An acne strain-selective antibacterial agent comprising at least one fatty acid selected from the group consisting of saturated fatty acids having 14 to 20 carbon atoms (C14:0 to C20:0) and a monounsaturated fatty acid having 18 carbon atoms (C18:1) in a free form or a salt form, or an ester thereof.

2. The acne strain-selective antibacterial agent according to claim 1, wherein the fatty acid is at least one selected from the group consisting of myristic acid, pentadecanoic acid, palmitic acid, heptadecanoic acid, stearic acid, oleic acid, vaccenic acid, and arachidic acid.

3. The antibacterial agent according to claim 1 or 2, wherein the fatty acid is at least one selected from the group consisting of myristic acid, pentadecanoic acid, palmitic acid, and heptadecanoic acid.

4. The antibacterial agent according to any one of claims 1 to 3, wherein the fatty acid comprises at least pentadecanoic acid.

5. The antibacterial agent according to any one of claims 1 to 4, wherein the ester comprises at least a monoacylglycerol.

6. The antibacterial agent according to any one of claims 1 to 5, wherein the ester is a monoacylglycerol, a diacylglycerol, a triacylglycerol, a cholesterol ester, or a wax ester, or a mixture thereof.

7. A skin-care composition comprising the antibacterial agent according to any one of claims 1 to 6, and a carrier acceptable for skin application.

8. The composition according to claim 7 for treatment or prevention of acne.

9. The composition according to claim 8 for pharmaceutical, cosmetic, or clothing use.

10. Use of at least one fatty acid selected from the group consisting of saturated fatty acids having 14 to 20 carbon atoms (C14:0 to C20:0) and a monounsaturated fatty acid having 18 carbon atoms (C18:1) in a free form or a salt form, or an ester thereof, for producing an acne strain-selective antibacterial agent or a skin-care composition.
